(19) Europäisches Patentamt
European Patent Office
Office européen des brevets

(11) Numéro de publication: **0 007 258**
**A1**

(12) **DEMANDE DE BREVET EUROPEEN**

(21) Numéro de dépôt: **79400380.6**

(22) Date de dépôt: **12.06.79**

(51) Int. Cl.³: **C 07 D 401/04**
**A 61 K 31/40**

(30) Priorité: **29.06.78 US 920598**

(43) Date de publication de la demande:
**23.01.80 Bulletin 80/2**

(84) Etats Contractants Désignés:
**BE CH DE FR GB IT LU NL SE**

(71) Demandeur: **PHARMINDUSTRIE**
**35 Quai du Moulin de Cage**
**F-92231 Gennevilliers(FR)**

(72) Inventeur: **Uzan, André Victor**
**35, Avenue Victor-Hugo**
**F-75016 Paris(FR)**

(74) Mandataire: **Houssin, Jean et al,**
**PRODUITS CHIMIQUES UGINE KUHLMANN Service**
**Propriété Industrielle Tour Manhattan Cedex 21**
**F-92087 Paris La Defense 2(FR)**

(54) **Dérivés de l'indole et leur utilisation comme anxiolytiques.**

(57) Utilisation des composés de formule:

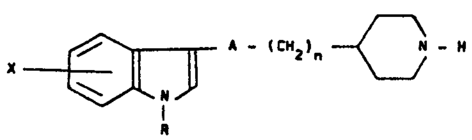

(I)

dans laquelle R représente un atome d'hydrogène, un groupe alkyle ayant de 1 à 4 atomes de carbone ou un groupe aralkyle dans lequel le groupe alkyle contient de 1 à 2 atomes de carbone; X représente un atome d'hydrogène ou d'halogène, un groupe alkyle, alkoxy ou alkylthio, le groupe alkyle contenant de 1 à 4 atomes de carbone; A représente -CO- ou $-CH_2-$; n'étant égal à 1 ou 2, et de leurs sels pharmaceutiquement acceptables dans le traitement de l'anxiété, à l'exception des méthodes de traitement anxiolytique.

EP 0 007 258 A1

Croydon Printing Company Ltd

Dérivés de l'indole et leur utilisation comme anxiolytiques

L'activité anxiolytique des benzodiazépines en clinique humaine a déjà été reconnue. Parmi celles-ci on peut citer en particulier le diazepam.

Squires et Braestrup ont mis en évidence (Nature 266, 732 ; 1977 - Science 198, 849 ; 1977 - Nature 266, 678 ; 1977) l'existence dans le système nerveux central des mammifères de sites spécifiques se liant avec une forte affinité avec le diazepam tritié ($^3$H-diazepam). Ces sites représentent, semble-t-il, un nouveau type de récepteurs spécifiques des benzodiazépines (Leslie L. Iversen, News and Views, Nature 275, 477 ; 1978).

Ainsi qu'il a été établi (Braestrup et Squires, European Journal of Pharmacology 48, 1978, pages 263 à 270, en particulier pages 268 et 269), il existe des relations hautement significatives entre les affinités des diverses benzodiazépines pour le site de la liaison du $^3$H-diazepam in vitro et leurs activités dans les tests pharmacologiques révélateurs des effets anxiolytiques chez l'homme. Ces corrélations suggèrent très fortement que la liaison in vitro avec les membranes cérébrales reflète la liaison in vivo des benzodiazépines avec les sites récepteurs qui développent les effets pharmacologiques ; il semble admis que le site de la liaison du $^3$H-diazepam représente les récepteurs physiologiquement actifs, capables de capter la benzodiazépine in vivo.

L'affinité des benzodiazépines pour le site de la liaison du $^{3}H$-diazepam a fait l'objet de mesures. Ces mesures visent à exprimer la capacité d'une benzodiazépine à déplacer le $^{3}H$-diazepam du site de sa liaison. Elle s'exprime par la relation :

$$K_i = IC_{50} \left[ (1 + C) \quad K_D \right]$$

dans laquelle :

$K_i$ est exprimé en micro-moles (µM),
C représente la concentration en $^{3}H$-diazepam,
$K_D$ représente la constante d'affinité (2,74 µM),
$IC_{50}$ représente la concentration provoquant l'inhibition 50 % de la fixation du $^{3}H$-diazepam.

En dehors des benzodiazépines, Braestrup et Squires ont examiné une centaine de composés appartenant à vingt-deux classes pharmacologiques. Ils n'en trouvèrent aucun présentant une affinité remarquable pour le site de la liaison du $^{3}H$-diazepam, leur $K_i$ étant supérieur à 0,1 mM ou 100 µM. On pouvait donc penser que les sites récepteurs du $^{3}H$-diazepam réagissaient exclusivement avec les benzodiazépines. Or il a été trouvé, conformément à la présente invention, que des composés structurellement différents des benzodiazépines ont une forte affinité pour ces sites, leur $K_i$ étant inférieur à 100 µM. Ils peuvent être représentés par la formule générale :

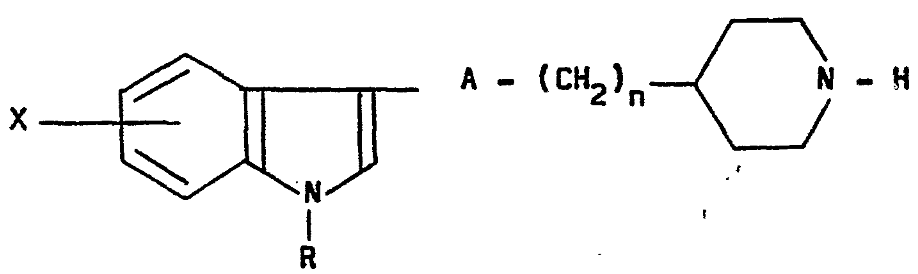

(I)

dans laquelle R représente un atome d'hydrogène, un groupe alkyle ayant de 1 à 4 atomes de carbone ou un groupe aralkyle dans lequel le groupe alkyle contient de 1 à 2 atomes de carbone ; X représente un atome d'hydrogène ou d'halogène, un groupe alkyle, alkoxy ou alkylthio, le groupe alkyle contenant de 1 à 4 atomes de carbone ; A représente -CO- ou $-CH_2-$ ; n étant égal à 1 ou 2.

Les composés de formule (I) sont des composés chimiquement connus (J.I. de Graw, J. Heterocyclic Chemistry 1966, 3 (1) pages 67 à 69 ; brevet français 1693.M ; brevet anglais 1.126.245 ; brevet anglais 1.023.781 ; G. Tacconi Farmaco. Ed. Sc. 20, 1965, page 470 ; brevet français 75.38051 du 12 Décembre 1975 ; brevet USA 4.064.255 délivré le 20 Décembre 1977). Les voies d'accès aux composés cétoniques (A = -CO-) et leur réduction pour l'obtention des (indolyl-3)-méthyl-, éthyl- et propyl-4 pipéridines sont plus particulièrement décrites dans le brevet français 75.38051 et dans le brevet américain 4.064.255 précités. Ces deux publications ont fait connaître en outre que les composés de formule (I) sont avantageusement utilisables comme antidépresseurs. Cette application thérapeutique résultait de la réponse positive donnée par la méthode de Kannengiesser et la technique de Baus Lassens à la question de savoir si les composés de formule (I) pouvaient être des inhibiteurs de la recapture de la sérotonine (hydroxy-5 tryptamine) ou de la noradrénaline. Les antidépresseurs empêchent leur reprise par le neurone qui les a libérés dans la fente synaptique. Les composés de formule (I) se comportent ainsi, principalement à l'égard de la sérotonine (G. Le Fur et A. Uzan, Biochemical Pharmacology, vol. 26, pages 497-503, 1977).

Les benzodiazépines par contre ont pour effet, semble-t-il, de rehausser les actions de l'acide γ aminobutyrique au niveau de ses récepteurs. D'autre part, comme Squires et Braestrup l'ont mis en évidence, elles se fixent sur les sites récepteurs privilégiés du $^{3}H$-diazepam (Leslie L. Iversen, News and Views, Nature vol. 275, page 477).

Il apparaît ainsi que les fonctions du système nerveux central, qui doivent être corrigées en vue du traitement de l'anxiété, sont différentes de celles qui sont mises en jeu dans le traitement de la dépression. Les sites du système nerveux central, au niveau desquels s'exercent ces fonctions sont également différents.

D'autres travaux ont d'ailleurs démontré la validité de la distinction entre les états d'anxiété et les maladies dépressives (T.A. Kerr, M. Roth, etc... British J. Psychiat. 1974, 124, pages 125-133). Cette distinction est reprise par la Food and Drug Administration (Psychopharmacology Bulletin, vol. 14, N° 2, 1978, page 45 ; Guidelines for the Clinical evaluation of Antidepressant Drugs, Sept. 1977 ; Guidelines for the Clinical evaluation of Antianxiety Drugs, Sept. 1977, U.S. Department of Health).

Ainsi qu'il a été trouvé conformément à la présente invention les composés de formule (I) possèdent une forte affinité pour les sites récepteurs des benzodiazépines et sont capables de déplacer le $^{3}$H-diazepam de ses sites de fixation. Cette aptitude a été mise en évidence in vitro par mesure de l'inhibition de la liaison spécifique du $^{3}$H-diazepam dans la cervelle du rat suivant la méthode de Möhler et al. (Life Sci. 20, 1977, page 2101).

On prélève par dissection le cortex cérébral de rats Charles River pesant en moyenne 150 g. Les tissus sont homogénéisés à l'aide d'un potter Braun Melsungen dans 20 volumes de solution de saccharose 0,32 M préalablement refroidie dans un bain de glace.

L'homogénat est centrifugé à 1.000 g durant 10 minutes à + 4°C. Le surnageant est séparé puis centrifugé à 20.000 g durant 20 minutes. Le culot de centrifugation est remis en suspension dans un tampon Krebs-Tris pH 7,4 dont la composition est la suivante : NaCl 118 mM, KCl 4,8 mM, $CaCl_2$ 1,2 mM,

$MgCl_2$ 1,2 mM, Tris 15 mM, HCl q.s.p. pH 7,4. Cette suspension est utilisée pour la mesure de la liaison spécifique du diazepam tritié. On y dose les protéines selon la méthode de Lowry et coll. (J.Biol.Chem., 193, 265, 1951). La mesure de la liaison du diazepam tritié est réalisée par incubation de parties aliquotes de la suspension (volumes correspondant à 1 ou 2 mg de protéines) avec du diazepam tritié, en l'absence ou en présence de concentrations élevées de diazepam non marqué. L'incubation est réalisée à + 4°C durant 15 minutes dans un volume de 2 ml de tampon Krebs-Tris pH 7,4 contenant 1,5 nM de diazepam tritié (méthyl-1 $^3H$-diazepam en provenance de New England Nuclear : activité spécifique 79,87 Ci/mMole), en l'absence ou en présence de diazepam non marqué (10 µM). La réaction est arrêtée par filtration sous vide sur filtre de fibres de verre Whatman GF/C. Les filtres sont lavés à deux reprises avec chaque fois 5 ml de tampon Krebs-Tris pH 7,4, puis solubilisés dans 10 ml d'Instagel® (Packard). Chaque essai comporte un "blanc" permettant de vérifier qu'il n'y a pas d'adsorption de radioactivité sur le filtre seul. Le diazepam tritié qui se lie en présence de 10 µM de diazepam non marqué représente la liaison non spécifique. Celle-ci est déduite du diazepam tritié lié en l'absence de diazepam non marqué représentant la liaison totale. La différence correspond à la quantité de diazepam tritié liée spécifiquement. Dans les conditions expérimentales décrites (1,5 nM de diazepam tritié) la liaison non spécifique représente seulement 2 à 5 % de la liaison totale.

Dans le tableau suivant les exemples 1' et 8' à 14' sont ceux du brevet américain 4.064.255 ou du brevet français 75.38051 donnant la préparation des diverses pipéridines.

- TABLEAU I -

| Inhibition de la liaison du $^{3}H$-diazepam avec les membranes de la cervelle du rat | | |
|---|---|---|
| Ex. | PRODUITS | $K_i$ (en µM) |
| 1 | Indolyl-3-(piperidyl-4-methyl)cetone (Exemple 1') | 43 |
| 2 | [(Methoxy-5 indolyl-3)-2 éthyl]-4 piperidine (Exemple 8') | 40 |
| 3 | [(Methyl-1 indolyl-3)-2 éthyl]-4 piperidine (Exemple 9') | 90 |
| 4 | [(Indolyl-3)-2 éthyl]-4 piperidine (Exemple 10') | 64 |
| 5 | (Indolyl-3 methyl)-4 piperidine (Exemple 11') | 90 |
| 6 | [(Chloro-5 indolyl-3)-2 éthyl]-4 piperidine (Exemple 12') | 37 |
| 7 | [(Indolyl-3)-3 propyl]-4 piperidine (Exemple 13') | 70 |
| 8 | [(Benzyl-1 indolyl-3)-2 éthyl]-4 piperidine (Exemple 14') | 33 |
| | Imipramine | non active |

L'efficacité des composés de formule (I) dans le test de Möhler n'est pas en corrélation avec une efficacité dans les tests de comportement tel que le test de la bataille électrique chez la souris, dans lequel les benzodiazépines sont actives (cf. Tedeschi, R.E. et al, J. Pharmacol., 125 (1959), 28). Cette différence confère aux produits selon l'invention un spectre entièrement nouveau d'activité, à savoir une activité anxiolytique sans propriétés sédatives.

Propriétés toxicologiques.

Le tableau II suivant donne les valeurs déjà données dans le brevet américain 4.064.255 ou le brevet français 75.38051 et fournit des données supplémentaires obtenues suivant les mêmes tests.

- TABLEAU II -

| PRODUITS | I.V. | Toxicité aigue chez la souris $DL_{50}$ (mg/kg) |
| --- | --- | --- |
| | | Voie orale |
| Exemple 1 | 47 | entre 300 et 900 |
| Exemple 2 | 41 | 675 |
| Exemple 3 | 44 | 225 |
| Exemple 4 | 60 | 600 |
| Exemple 5 | 57 | 225 |
| Exemple 6 | 85 | 625 |
| Exemple 7 | 29 | 600 |
| Exemple 8 | 33 | 525 |

Applications thérapeutiques.

Les produits de formule (I) sont utiles en thérapeutique humaine dans les états d'anxiété résultant d'une tension ou agitation ou de facteurs émotionnels (appréhension, agoraphobie, troubles de la personnalité). A la différence des benzodiazépines qui sont bien connues comme anxiolytiques et sédatifs, les composés de formule (I) et leurs sels pharmaceutiquement acceptables, sont dépourvus d'effets sédatifs. Ils agissent comme stimulants psychiques. Ils peuvent être administrés oralement ou par voie parentérale. La posologie recommandée va de 50 mg à 300 mg de substance active par jour, soit en dose unique, soit en doses séparées de préférence, allant de 1 à 50 mg par kg.

Formulation - Préparation des compositions - Conditionnement.

En vue de son application thérapeutique, le composé pipéridylindol peut être présenté sous forme de comprimé nu ou enrobé, de gélule, de solution injectable pour administration parentérale.

Les exemples ci-dessous ne sont pas limitatifs, tant en ce qui concerne les doses, formes, compositions, procédés de fabrication.

FORME COMPRIME

Les doses sont de 10, 25, 50 mg par comprimé sans être limitatives.

- Formules

| | | | |
|---|---|---|---|
| Pipéridyl-indol................ | 10 mg | 25 mg | 50 mg |
| Cellulose microcristalline..... | 20 mg | 75 mg | 75 mg |
| Mannitol....................... | 50 mg | 41 mg | 41 mg |
| Polyvidone excipient.......... | 4 mg | 10 mg | 10 mg |

| | | | |
|---|---|---|---|
| Caséine méthylée.............. | 10 mg | 0 | 0 |
| Carboxyméthylamidon sodique.. | 0 | 25 mg | 25 mg |
| Silice colloïdale............ | 0 | 4 mg | 4 mg |
| Talc........................ | 5 mg | 18 mg | 18 mg |
| Stéarate de magnésium........ | 1 mg | 2 mg | 2 mg |
| Pour un comprimé | 100 mg | 200 mg | 225 mg |

Le pipéridyl-indol après tamisage est mélangé soigneusement dans un mélangeur planétaire au mannitol et à la cellulose microcristalline. Le mouillage destiné à l'obtention d'une masse granulable est effectué à l'aide d'une solution méthanolique de polyvidone à 20 %. Compléter le mouillage si nécessaire à l'aide de méthanol. Granuler la masse humide sur granulateur oscillant équipé d'une grille de 3 mm d'ouverture de maille. Sécher le granulé à 45°C dans une étuve ventilée. Calibrer le granulé sec sur tamis de 1 mm d'ouverture de maille. Ajouter le reste des adjuvants et mélanger soigneusement au mélangeur planétaire par exemple. Comprimer à l'aide d'une presse alternative ou rotative. Les poids des comprimés sont respectivement de 100, 200 et 225 mg pour les doses suivantes : 10, 25, 50 mg.

Les caractéristiques des comprimés sont :

| | 10 mg | 25 mg | 50 mg |
|---|---|---|---|
| Poids | 100 mg | 200 mg | 225 mg |
| Dureté (Duromètre RWK) | 4 kg | 8 kg | 10 kg |
| Diamètre | 6-R 7 | 8-R 7 | 9-R 8 |
| Epaisseur (mm) | 3,2-3,3 | 4,1-4,2 | 4 |
| Délitement (mn) | 10 | 10 | 10 |

- <u>Conditionnement</u>

Les comprimés sont conditionnés en piluliers munis d'une cape en polyéthylène avec compensateur ou en blisters. Ces modes de conditionnement ne sont pas limitatifs. Les comprimés

aux formules ci-dessus ont une bonne stabilité à la température ambiante de 20°C.

FORME GELULE

Les granulés obtenus selon les formules des comprimés peuvent être mis en gélules. A titre d'exemple le granulé prêt à être comprimé au dosage 25 mg est mis en gélules.

- Formule

| | |
|---|---|
| Pipéridyl-indol............... | 25 mg |
| Cellulose microcristalline.... | 75 mg |
| Mannitol...................... | 41 mg |
| Polyvidone excipient.......... | 10 mg |
| Carboxyméthylamidon sodique... | 25 mg |
| Talc.......................... | 18 mg |
| Stéarate de magnésium......... | 2 mg |
| Silice colloïdale............. | 4 mg |
| Pour une gélule taille n° 2 | 200 mg |

La stabilité des gélules est identique à celle des comprimés.

- Conditionnement

Les gélules sont conditionnées en piluliers ou en blisters.

SOLUTE INJECTABLE

- Formule

| | |
|---|---|
| Pipéridyl-indol...................... | 25 mg |
| Acide ascorbique..................... | 25 mg |
| Métabisulfite de sodium.............. | 2,5 mg |
| Chlorure de sodium................... | 11 mg |
| Eau pour préparation injectable q.s.p. | 2 ml |

- Préparation

Faire dégager l'oxygène dissous dans l'eau pour préparation injectable par barbotage d'azote. Toujours sous courant d'azote, dissoudre successivement l'acide ascorbique, le pipéridyl-indol, le métabisulfite de sodium et le chlorure de sodium.

Compléter par de l'eau dégazée au volume requis. Filtrer la solution sur filtre Millipore à 0,22 µ. Répartir en ampoules de 2 ml sous couverture d'azote. Sceller les ampoules et les stériliser à 100° pendant 1 heure.

Aspect général : soluté limpide, incolore
pH : 4,8
Δt cryoscopique : -0,60°C

Le soluté stérile est stable après 6 mois à 37°C et 6 mois à 45°C.

- Conditionnement

En boîtes de 1, 6, 10 ou 20 ampoules selon usage.

Revendications de brevet

1. Composés pharmacologiquement actifs possédant de l'affinité pour les sites récepteurs du $^3H$-diazepam caractérisés en ce qu'ils sont constitués par les dérivés indoliques de formule :

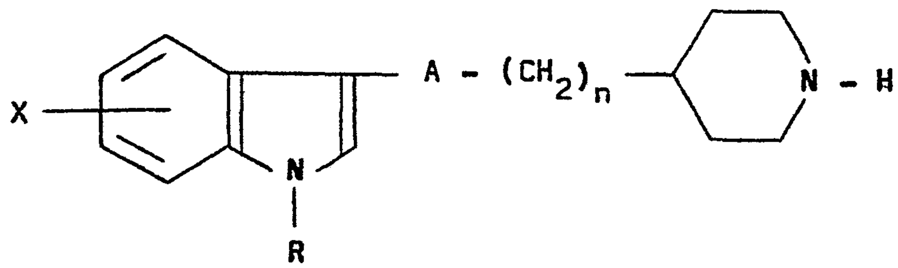 (I)

dans laquelle R représente un atome d'hydrogène, un groupe alkyle ayant de 1 à 4 atomes de carbone ou un groupe aralkyle dans lequel le groupe alkyle contient de 1 à 2 atomes de carbone ; X représente un atome d'hydrogène ou d'halogène, un groupe alkyle, alkoxy ou alkylthio, le groupe alkyle contenant de 1 à 4 atomes de carbone ; A représente -CO- ou $-CH_2-$ ; n étant égal à 1 ou 2.

2. Composés tels que revendiqués sous 1) dont le $K_i$ est inférieur à 100 µM.

3. Utilisation des composés définis sous 1) et de leurs sels pharmaceutiquement acceptables dans le traitement de l'anxiété, à l'exception des méthodes de traitement anxiolytique.

0007258

Numéro de la demande

Office européen des brevets

**RAPPORT PARTIEL DE RECHERCHE EUROPEENNE**

qui selon la règle 45 de la Convention sur le brevet européen est considéré, aux fins de la procédure ultérieure, comme le rapport de recherche européenne

EP 79 40 0380

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.³) |
|---|---|---|---|
| DX | FR - A - 2 334 358 (SOGERAS)<br>* Demande complète *<br>---- | 1-3 | C 07 D 401/04<br>A 61 K 31/40 |
| | | | DOMAINES TECHNIQUES RECHERCHES (Int. Cl.³) |
| | | | C 07 D 401/04 |

## RECHERCHE INCOMPLETE

La division de la recherche estime que la presente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes:
Revendications ayant fait l'objet de recherches incomplètes:
Revendications n'ayant pas fait l'objet de recherches: 3
Raison pour la limitation de la recherche:

Méthode de traitement chirurgical thérapeutique du corps humain ou animal
(Article 52(4) de Convention sur le brevet européen).

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

| Lieu de la recherche | Date d'achèvement de la recherche | Examinateur |
|---|---|---|
| La Haye | 13-09-1979 | MAISONNEUVE |

OEB Form 1505.1 06.78